Europäisches Patentamt

European Patent Office

Office européen des brevets

(11) Publication number: **0 389 457**
**A2**

# EUROPEAN PATENT APPLICATION

(21) Application number: 90850099.4

(22) Date of filing: **15.03.90**

(51) Int. Cl.5: **C12N 9/02, C12N 1/20, C12Q 1/26**

(30) Priority: **20.03.89 SE 8900982**

(43) Date of publication of application:
**26.09.90 Bulletin 90/39**

(84) Designated Contracting States:
**AT BE CH DE DK ES FR GB GR IT LI LU NL SE**

(71) Applicant: TECHNOLOGICAL INSTITUTE OF ICELAND
Keldnaholt
IS-110 Reykjavik(IS)

(72) Inventor: **Baldursson, Sigurdur**
**Digranesvegur 72**
**IS-200 Kopavogur(IS)**
Inventor: **Kristjansson, Jakob K.**
**Skipasund 66**
**IS-104 Reykjavik(IS)**

(74) Representative: **Bergvall, Stina-Lena et al**
**Dr. Ludwig Brann Patentbyrä AB Box 7524**
**Kungsgatan 3**
**S-103 92 Stockholm(SE)**

(54) Isolation of a novel thermophilic bacterial species thermus anaerobicus, production and preparation of a novel formate linked nitrate reductase enzyme system from it for analytical and commercial use.

(57) A novel thermostable formate linked nitrate reductase enzyme system is produced from a novel strain of the genus *Thermus*. The enzyme system is suitable for determination of nitrate down to the concentration of 0,005 ppm NO3-N. The reduction of nitrate is suitable for this system in boiling water. The reduction of nitrate to nitrite takes 10 minutes to complete in boiling water and is not inhibited by oxygen. For this reduction a whole cell preparation of *Thermus anaerobicus*, or a partial purification thereof, is used. A conventional color indicator system is used to quantify nitrite.

EP 0 389 457 A2

# ISOLATION OF A NOVEL THERMOPHILIC BACTERIAL SPECIES THERMUS ANAEROBICUS, PRODUCTION AND PREPARATION OF A NOVEL FORMATE LINKED NITRATE REDUCTASE ENZYME SYSTEM FROM IT FOR ANALYTICAL AND COMMERCIAL USE

## BACKGROUND OF THE INVENTION

This invention relates to a novel thermophilic microorganism capable of producing thermostable formate linked nitrate reductase enzyme system, a method for isolating and culturing the microorganism and a method for producing the formate linked nitrate reductase enzyme system and uses thereof analytically and commercially, for the quantification of nitrate in nitrate containing samples.

Formate linked nitrate reductase enzyme system has previosly been detected in some mesophilic microorganisms (Lowe and Gillespie, 1975). Those enzyme systems are not thermostable and are quite unstable at room temperature. They have limited diagnostic use and no commercial use as they lack sufficent stability, are inhibited by presence of oxygen and reduction of nitrate is not quantitative in a conventional time for analytical purposes.

It is generally known that there is a need for a quick and convenient analysis for nitrate in water and wastewater. The inventors have isolated a novel microorganism from hot springs in Iceland by in situ enrichment which organism is capable of producing a novel thermostable formate linked nitrate reductase enzyme system.

The thermostable formate linked nitrate reductase enzyme system of the present invention, can react at elevated temperatures in presence of oxygen and complete a reduction of nitrate to nitrite in a very short time. The formate linked nitrate reductase enzyme system is stable in solution at room temperature for weeks and in dried form for months.

Commercial products available today, for the determination of nitrate, make use of cadmium to reduce nitrate to nitrite and can detect nitrate down to 0.010 ppm NO3-N. The present invention can quantify nitrate down to 0.005 ppm NO3-N, is much easier to use than the cadmium reduction method and does not pose a pollution problem as the disposal of cadmium does.

It is an objective of this invention to provide for the preparation and use of thermostable formate linked nitrate reductase enzyme system from thermophilic microorganisms, such as strains of the new species *Thermus anaerobicus* which are facultative anaerobes; and use whole cells or partial purification therof for analytical and commercial use for the quantification of nitrate. Other species such as *Thermus aquaticus* do not have these enzyme systems and are strict aerobes.

The invention will hereinafter be described with respect to the isolation of the above mentioned bacteria and the preperation of the thermostable formate linked nitrate reductase enzyme system using for example *Thermus* like strains isolated from Icelandic hot springs as representative of the thermophilic microorganisms, and a description of quantitative determination of nitrate with the above mentioned enzyme system.

## SUMMARY OF THE INVENTION

Thermophilic bacteria contain thermostable enzymes witch are stable and often inactive at room temperatures but become active upon heating to high temperatures such as 60-100 °C. These instrinsic properties of the enzymes make them useful as catalysts as they have long shelf life and they do not have to be kept at low temperatures for storage. The novel thermophilic bacterial strains of. *T. anaerobicus* isolated according to the present invention have novel formate linked nitrate reductase enzyme system, which are active at temperatures up to 100 °C at pH 4,5; are useful in reducing and quantifying nitrate in a short time and in an easy way.

The invention relates to a method for preparation of a novel formate linked nitrate reductase enzyme system from thermophilic facultative anaerobic bacteria, the analytical and commercial use thereof, especially of quantifying nitrate in samples containing nitrate, such as water, wastewater and where nitrate can be extracted into water such as vegetables and other foodstuffs.

The thermostable enzyme system are prepared by inoculating a substrate enriched basal medium with a strain of thermophilic bacteria, incubation of the bacterial cells at an elevated temperature, collecting and washing of the cells. The cells which contain formate linked nitrate reductase enzyme system are used to reduce nitrate to nitrite in the presence of formate and a buffer substance.

Within the scope of analytical chemistry, the quantitative determination of nitrate plays an especially important part in chemical diagnosis. The nitrate concentration in drinking water, wastewater and some foodstuffs especially vegetables, pose a serious health and pollution problem.

In comparision with purely chemical or partly enzymatic or spectrophotometric determinations (e.g. the methods used especially frequently with the use of cadmium, the Devards-alloy reduction method, the spectrophotometric screening method (APHA, 1985) the present invention is more specific and easier to use. The important advantages in comparision with the above chemical methods is also the avoidance of the use of corrosive, strongly acidic or toxic reagents, the universal usability for carrying out the method manually and not only for skilled labor, but also for a non skilled person as well as the possibility to be able to evaluate the measured results within 20 minutes after collecting the sample i.e. from a water source far away from laboratories by comparing the formed color to a standard chromatographic chart.

Hitherto, comparable enzyme systems known for determination of nitrate are inhibited by oxygen, lack thermal stability or the reaction takes place extraordinarily slowly, so that the reactiom time is beetween 4-5 hours at 45 °C. It is therefore, surprising that the reduction of nitrate to nitrite according to the invention can, nevertheless, be carried out in 10 minutes even in presence of oxygen.

Thermophilic processes have the advantage of higher reaction rates and the time for conversion of substrates into products is shorter. Thermophilic processes in boiling water are specially important because temperature control of the heating process is not needed. Another intrinsic property of importance is low activity and high stability of thermophilic cells and enzymes at room temperature, which lessen the need for refrigeration during cell and enzyme recovery, and during storage and a catalytic activity with a longer half life is provided for commercial use.


## BRIEF DESCRIPTION OF THE DRAWINGS

Fig. 1 shows the dependence of A against the concentration of nitrate in the sample.
Fig. 2 shows how pH influence reaction rate.
Fig. 3 shows how temperature influence reaction rate.
Fig. 4 shows temperature inactivation curves.


## DETAILED DECRIPTION OF THE INVENTION

The bacterial strains of this invention have the following *Thermus* characters: Gram negative; do not form spores; oxidase positive; thin rods a few $\mu$m long; forming filaments at 75 °C in liquid culture: One character that differentiates from the typical *Thermus* spp. is anaerobic growth with nitrate as electron acceptor. Strains of Thermus, which can grow anaerobically on nitrate and reduce nitrate to nitrite have been decribed before but they have not been characterized in details. They have not been shown to be able to use formate as reductant for nitrate nor have any characteristics of their reductase been determined (Hudson et al., 1989; Munster et al., 1986; Sharp and Williams, 1988) Thermostable formate linked nitrate reductase enzyme system has not been described before. Therefore this is a novel microorganism with a novel reductase enzyme system. Based on these new characters the isolates are defined as a new species, *Thermus anaerobicus.*

The *T. anaerobicus* strains used in this invention and having the same characters are: M-10 (DSM-5225) and M-41 (DSM-5226).

The strains were isolated from an 3 months old *in situ* enrichment, in a hot spring in Iceland at 75 °C fed with KNO$_3$ solution. The isolation procedure was as follows: samples from the *in situ* enrichment were taken and incubated in completely filled bottles for 2 weeks at 75 °C and then inoculated again to completely filled bottles with fresh media and incubated for two weeks. Then samples from the bottles were filtered on an 0.45 $\mu$m filter and the filter were placed on an agar medium. This was incubated for two days at 65 °C and colonies on the filter were picked and streaked on agar plates until a pure culture was established.

For the growth of the cells, use was made of the following: fermentation for cell growth was performed in complex medium that contained 0.5 % yeast extract and 0.5 % KNO$_3$ by weight. For agar medium, 3 % agar (30 g/l) were used. The pH was set at about neutral before autoclaving the medium (see example 1).

The cells were cultured in 500 ml flasks containing 400 ml medium and were incubated at 65 °C or 75 °C without shaking. Thereafter, the cells were grown in 150 liter fermentor containing 100 liters of the same medium as described above.

For preperation of cells and enzymes, cells were harvested by centrifugation and washed in 0.85 NaCl solution until nitrate and nitrite free (see example 2). After washing, the cells were repeatedly frozen and thawed and diluted to a concentration (wet weight/ml) so that 0.03 ml of this enzyme solution (see example 3) will quantitatively reduce 0.005 to 0.75 $\mu$g $NO_3$-N in 10 minutes per assay tube under the standard assay conditions. Important improvements gained from this method are the possibility to use formate, which is inexpensive and a non-toxic source of reductant and no need to avoid oxygen in the assay as was neccessary with previously described system using a non-thermostable enzyme. The method for using this enzyme preperation for quantification of nitrate in nitrate containing samples is described in example 4.

EXAMPLE 1. Growth of *Thermus anaerobicus*.

The basal salts medium used for culture of *T. anaerobicus* is as follows:

| Nitrilotriacetic acid | 45 mg/l |
|---|---|
| $FeCl_3$ x $4H_2O$ | 350 $\mu$g/l |
| $MnCl_2$ x $4H_2O$ | 175 $\mu$g/l |
| $CoCl_2$ x $6H_2O$ | 105 $\mu$g/l |
| $ZnCl_2$ | 70 $\mu$g/l |
| $CuCl_2$ x $2H_2O$ | 18 $\mu$g/l |
| $NaMoO_4$ x $2H_2O$ | 245 $\mu$g/l |
| $NaSeO_3$ x $5H_2O$ | 280 $\mu$g/l |
| $H_3BO_4$ | 7 $\mu$g/l |
| $NiCl_2$ x $6H_2O$ | 7 $\mu$g/l |
| $KNO_3$ | 5g/l |

Buffer is 20 mM phosphate buffer pH 7.2. To one liter medium is added 5g yeast extract or 2.5 g yeast extract and 2.5g tryptone. For growth in 500 ml flasks $NaN_3$, 1 mM, is added before autoclaving. When grown in 1501 fermenter $NaN_3$, 1 mM, is added after initial aerobic growth of the cells.

The 500 ml flasks are incubated for 4 days at 65 °C in an incubator without shaking and cells are harvested and used in assays for nitrate.

The 100 l fermentor is inoculated with 2 l of 18 hours old, aerobically grown cells. Constant stirring at 250 rpm and continuous air gassing (5 l/min) at 65 °C was maintained for 7 hours, then the culture medium was continuously gassed with $N_2$ (1 l/min) for 20 hours at 65 °C and the cells are harvested with centrifugation and wased in 0.85 % NaCl until nitrate and nitrite free.

EXAMPLE 2. Production of whole cell suspension.

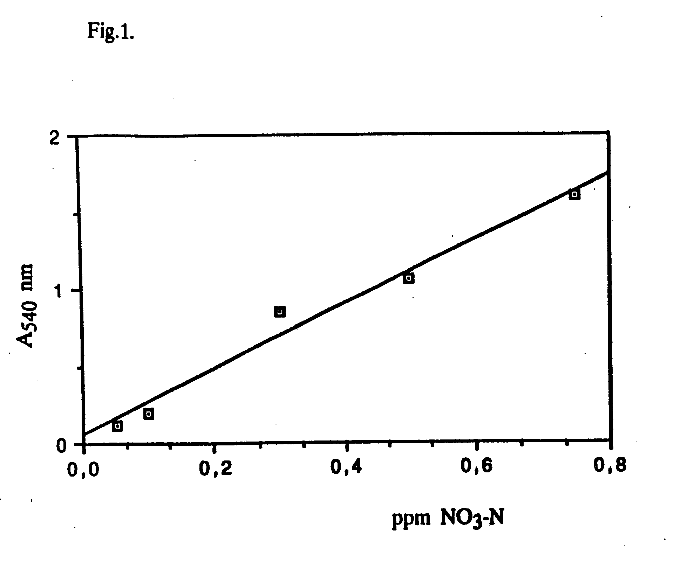

Washed whole cells are suspended in the following solution: 40 % glycerol, 0.85 % NaCl and 0.17 % sodium benzoate. The activity of this enzyme solution will reduce 0.005 to 0.75 ppm $NO_3$-N in a linear manner (see Fig 1). A typical concentration of wet cells in the suspension is 0.1 g/ml.

EXAMPLE 3. Characterization of formate linked nitratereductase enzyme system.

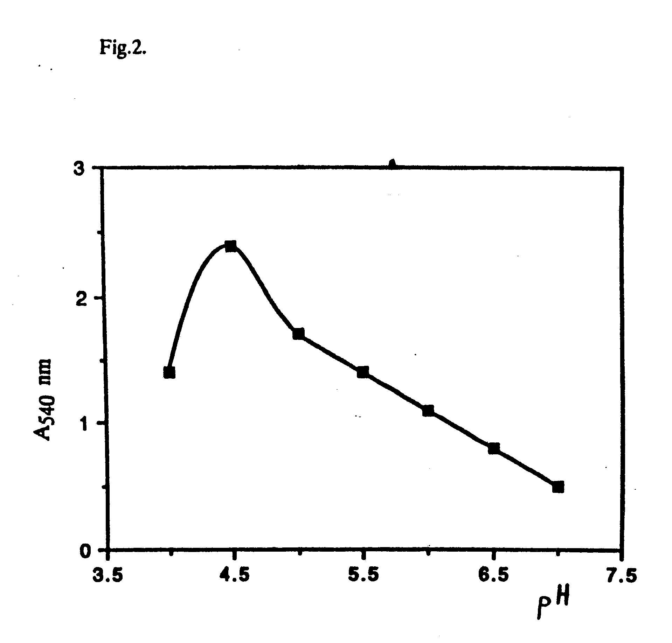

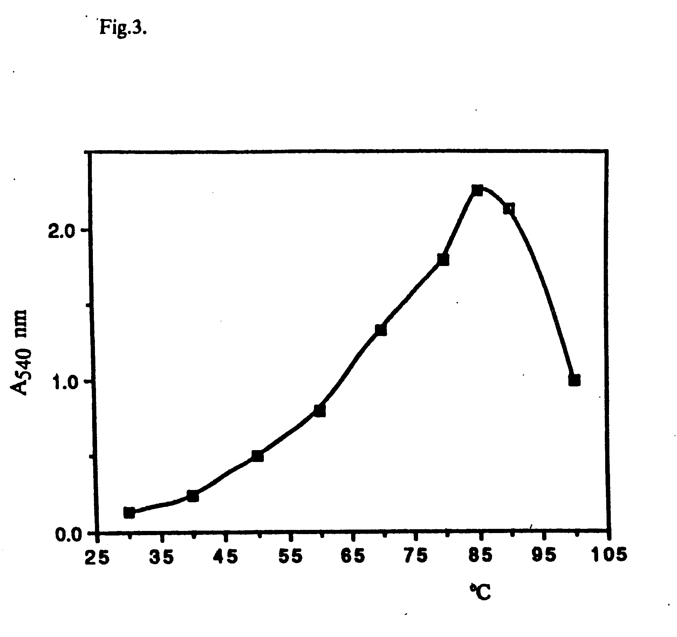

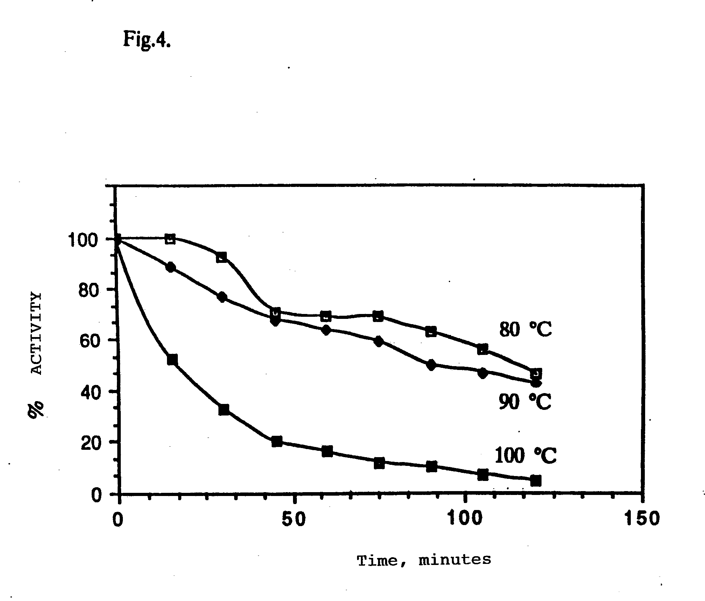

The enzymatically active whole cell preparation as prepared in Example 1 and 2 are assayed at 85 °C and pH 4.5 in 50 mM formate and 50 ppm $NO_3$-N if not otherwise stated. Optimal activity is at pH 4.5 (see Fig. 2). Maximum activity is at 85 °C (see Fig. 3). Half live in aqueus solution at assay conditions for nitrate (boiling water) is at least 15 min, at 90 °C at least 90 min, and at 80 °C at least 120 min (see Fig. 4).

EXAMPLE 4. Process for the quantitative determination of nitrate.

Solution A. Formate linked nitrate reductase enzyme system solution. The enzyme solution displays the catalytic activity of reducing 0.005 to 0.75 $\mu$g NO3-N in 1 ml sample solution in 10 minutes in boiling water and was stored as solution in 40 % glycerol and 0.85 % NaCl and 0.17 % sodium benzoate, with a whole cell concentration of 0.1 g/ml (wet weight/ml). This solution is used directly in the assay and can be kept at room temperature for 6 weeks.

Solution B: Formate solution. 1.0 M formate in citrate phosphate buffer pH 4,5.
Solution C: Sample solution containing 0.005 to 0.75 ppm $NO_3$-N.

COLOR REAGENT:

Solution D: 1% sulfanilic acid in 3 M HCl.
Solution E: 0.02% N-(1-Naphtyl)-ethylenediamine dihydrochloride.
Process for carrying out a test.
Measurement against reagent blank.
Into a 2 ml assay tube pipette:

|  | Sample value | Reagent blank |
|---|---|---|
| Destilled water without nitrite | - | 1.0 ml |
| Sample solution (C)[+] | 1.0 ml | - |
| Solution A | 30 $\mu$l | 30 $\mu$l |
| Solution B | 50 $\mu$l | 50 $\mu$l |
| Mix, incubate in boiling water for 10 min then add: | | |
| Solution D | 0.2 ml | 0.2 ml |
| Solution E | 0.2 ml | 0.2 ml |

[+] Aqueus solutions of nitrate with concentrations of 0.005 to 0.75 ppm $NO_3$ - N.

After 10 minutes compare the formed color to standardized chromatographic chart or measure formed color at 540 nm and subtract the reagent blank.

EXAMPLE 5. Process for the quantitative determination of nitrate in various samples.

The process is as in example 4 except that solution C is made of food exteacts. Food samples were weighted and mixed in 50 ml distilled water in a blender for 3 min and kept in half filled volumetric flasks in a boiling water bath for 10 min. Then water was added to the mark and the samples were filtered through Whatman no. 1 filter paper. Results are shown in table 1.

Table 1.

| Assay of nitrate in food extracts. | | | | | |
|---|---|---|---|---|---|
| Extracted food | Measured nitrate in the assay, ppm NO3-N | | Recovered | Recovery % | Total amount $\mu$gNO3-N/g fresh sample |
|  | Analysed | Added | | | |
| Tomatoes | 0.260 | 0.08 | 0.330 | 87 | 95 |
| Sausage | 0.145 | 0.08 | 0.225 | 100 | 100 |
| White cabbage | 0.340 | 0.08 | 0.415 | 94 | 442 |
| Chinese lettuce | 0.970 | 0.08 | 1.050 | 100 | 2368 |

**Cited references**

APHA, AWWA, WPCF (1985). Standard methods for the Examination of Water and Wastewater. 16th ed. Washington, D.C.

Hudson, J. A., Morgan, H. W. & Daniel, R. M. 1989. Numerical Classification of Thermus Isolates from Globally Distributed Hot Springs. Systematic and Applied Microbiology 11, 250-256.

Lowe, R. H. & Gillespie, M. C. 1975. An Escherichia coli Strain for Use in Nitrate Analysis. Journal of Agriculture and Food Chemistry 23, 783-785.

Munster, M. J., Munster, A. P., Woodrow, J. R. & Sharp, R.J. 1986. Isolation and Preliminary Taxonomic Studies of Thermus Strains Isolated from Yellowstone National Park, USA. Journal of General Microbiology 132, 1677-1683.

Sharp, R. J. & Williams, R. A. D. 1988. Properties of Thermus ruber Strains Isolated from Icelandic Hot Springs and DNA:DNA Homology of Thermus ruber and Thermus aquaticus. Applied and Environmental Microbiology 54, 2049-2053.

## Claims

1. Novel thermophilic microorganism capable of producing thermostable formate linked nitrate reductase enzyme systems, comprising a substantially pure form of *Thermus anaerobicus.*

2. The microorganism according to claim 1, comprising a substantially pure form of *Thermus anaerobcius* M-10 and M-41.

3. The microorganism according to claims 1 and 2, comprising a substantially pure form of *Thermus anaerobicus* M-10 (DSM-5226) and M-41 (DSM-5225).

4. A process for isolating *Thermus anaerobicus* in a substantially biologically pure form, which comprises isolating a sample from hot spring, fed with KNO₃ solution, containing Thermus anaerobicus, culturing the bacteria from said sample in completely filled bottles at temperature and growth conditions producing suitable yield, and isolating Thermus anaerobicus from said culture.

5. Thermostable formate linked nitrate reductase enzyme system in an enzymatically active form, which reduces nitrate to nitrite at temperatures of 60 °C up to boiling point of water using formate as substrate, said enzyme system being derived from Thermus anaerobicus.

6. An enzymatically active whole cell preperation of the thermostable formate linked nitrate reductase enzyme system in claim 4, having an activity half life of at least 120 minutes at 80 °C, at least 90 minutes at 90 °C, at least 15 minutes at 100 °C as measured using nitrate and formate as substrates, said enzyme system being derived from *Thermus anaerobicus* and having maximum activity at pH 4.5 and temperature of 85 °C.

7. A method for producing thermostable enzymes said method comprising the steps of:
   a) inoculating a culture medium with a strain of thermophilic bacteria;
   b) incubating said bacteria at a temperature between 65-85 °C;
   c) incubating said bacteria without oxygen;
   d) purifying the bacteria, from spent culture supernatant by centrifuging, which contain thermostable formate linked nitrate reductase enzyme system.

8. Process for the determination of nitrate, comprising reacting a nitrate containing sample with thermostable formate linked nitrate reductase enzyme system in the presence of formate to form nitrite and measuring formed nitrite as a measure of nitrate in said sample by conventional techniques.

9. Process according to claim 8, wherein said process is done in hot water preferably in boiling water and done without any special precautions to avoid presence of oxygen.

10. Process according to claim 8, wherein the thermostable formate linked nitrate reductase enzyme system having an activity half life of at least 120 minutes at 80 °C., at least 90 minutes at 90 °C., at least 15 minutes at 100 °C., as measured using nitrate and formate as substrates, said enzyme system being derived from Thermus anaerobicus and having maximum activity at pH 4.5 and temperature of 85 °C.

11. Process according to claim 10, wherein the enzyme system is from Thermus anaerobicus M-10 (DSM-5226) and M-41 (DSM-5225).

12. The process of claim 8, wherein the thermostable formate linked nitrate reductase enzyme system is from bacteria of the genus Thermus.

Fig.1.

Fig.2.

EP 0 389 457 A2

Fig.3.

Fig.4.